Europäisches Patentamt

European Patent Office

Office européen des brevets

(19)

(11) Publication number: **0 034 846**
**B1**

# EUROPEAN PATENT SPECIFICATION

(12)

(45) Date of publication of patent specification: **02.05.84**

(51) Int. Cl.³: **A 61 K 7/08**

(21) Application number: **81200126.1**

(22) Date of filing: **03.02.81**

(54) Antidandruff lotion shampoo compositions.

(30) Priority: **07.02.80 US 119283**
**22.12.80 US 219281**

(43) Date of publication of application:
**02.09.81 Bulletin 81/35**

(45) Publication of the grant of the patent:
**02.05.84 Bulletin 84/18**

(84) Designated Contracting States:
**AT BE CH DE FR GB IT LI LU NL SE**

(56) References cited:
**BE - A - 671 930**
**CH - A - 430 058**
**FR - A - 2 060 407**
**FR - A - 2 133 805**
**GB - A - 1 280 671**
**US - A - 4 033 895**

(73) Proprietor: **THE PROCTER & GAMBLE COMPANY**
**301 East Sixth Street**
**Cincinnati Ohio 45202 (US)**

(72) Inventor: **Winkler, William Matthews**
**7271 Bobby Lane**
**Cincinnati, Ohio 45243 (US)**

(74) Representative: **Brooks, Maxim Courtney et al,**
**Procter & Gamble (NTC) Limited Whitley Road**
**Longbenton**
**Newcastle-upon-Tyne NE12 9TS (GB)**

Courier Press, Leamington Spa, England.

Antidandruff lotion shampoo compositions

The present invention is related to antidandruff lotion shampoo compositions which possess good lotion viscosity while also being quite stable with respect to separation.

Lotion compositions, particularly those containing particulate materials, traditionally suffer from liquid separation. If an attempt is made to avoid such separation the compositions become too viscous for satisfactory pouring. A totally acceptable product requires that both stability and viscosity be optimized.

The separate use of the components of the present invention in shampoos is known in the shampoo art.

U.S.—A—3,917,817 discloses a shampoo composition containing a piperazine based cationic polymer, 10% sodium alkyl sulfate, 4% lauryl monoethanolamide and 3% glycol distearate.

U.S.—A—4,013,787 discloses a similar composition. Japanese Application, with Open for Public Inspection number 60810, May 19, 1977 (Lion Fat & Oil), discloses shampoos containing 5% to 50% of an anionic surfactant, 1% to 10% of a fatty acid diethanol amide, 0.1% to 10% of an insoluble fine powder, and 1% to 10% of an ethyleneglycol ester.

U.S.—A—4,033,895 discloses a non-irritating shampoo containing 25% sodium lauryl sulfate (28% active), 2% montmorillonite, 2.1% zinc pyridinethione (48% active), 6% of a 7:3 mixture of lauric and myristic diethanolamides and 8% of stearyldimethylamide oxide (25% active). FR—A—2,133,805 discloses a shampoo containing 10% triethanolamine lauryl sulfate, 10% lauryldimethylamine oxide, 5% cocomonoethanolamide, 1.5% zinc pyridinethione and 3% diethylenetriamine.

While these references disclose compositions which contain components of the type present in the compositions of the present invention, they do not suggest combining the components in the amounts found critical by the present inventor.

It is an object of the present invention, therefore, to provide shampoos containing certain critical levels of surfactant, amide and a suspending agent as well as containing a particulate antidandruff agent.

It is a further object of the present invention to provide shampoos which have optimal viscosity and liquid separation.

These and· other objectives will become readily apparent from the detailed description which follows.

The present invention relates to antidandruff shampoo compositions comprising from 11% to 20% of an anionic surfactant, from 4% to 6% of a suspending agent, from 2% to 4% of an alkanol amide, a particulate antidandruff agent and water. The compositions exhibit very little liquid separation and preferably have a viscosity at 25°C. of from 2.5 Pa.s (2500 cps) to 6 Pa.s (6000 cps).

The compositions of the present invention comprise certain essential components and may additionally contain several optional components. Each of the components is discussed in detail below.

Anionic surfactant

The surfactant in the shampoos of this invention is anionic and is present at a level of from 11% to 20%, preferably 14% to 18%. Specifically, the surfactant is an alkyl sulfate, an ethoxylated alkyl sulfate or mixtures thereof. The alkyl sulfates found useful are the sodium, ammonium and triethanolamine alkyl sulfates having from 8 to 22 carbon atoms in the alkyl chain. Preferred are those sulfates obtained by sulfating the higher alcohols, those having from 8 to 18 carbon atoms.

The ethoxylated alkyl sulfates have alkyl chains of the type used with the alkyl sulfates but have from 1 to 6, preferably 3, ethoxy groups per molecule.

The most preferred surfactant is sodium alkyl sulfate. With this surfactant, as well as others, the viscosity may be controlled in the desired range by the total amount of buffering agent which may be present.

Suspending agent

The suspending agent useful in the present compositions can be any of several materials. Included are ethylene glycol esters of fatty acids having from 16 to 22 carbon atoms. Preferred are the ethylene glycol stearates, both mono and distearate. Other suspending agents found useful are alkanol amides of fatty acids, having from 16 to 22 carbon atoms, preferably 16 to 18 carbon atoms. Preferred alkanol amides are stearic monoethanolamide, stearic diethanolamide, stearic monoisopropanolamide and stearic monoethanolamide stearate.

Still other suitable suspending agents are alkyl (C$_{16-22}$) dimethyl amine oxides such as stearyl dimethyl amine oxide. Mixtures of these materials are also acceptable.

The suspending agent is present at a level of from 4% to 6%. The suspending agent serves to assist in suspending the antidandruff active and may give pearlescence to the product.

Amide

The amide used in the present compositions can be any of the alkanolamides of fatty acids known for use in shampoos. These are generally mono- and diethanolamides of fatty acids having from 8 to 14 carbon atoms. Preferred are coconut monoethanolamide, lauric diethanolamide and mixtures thereof.

The amide is present at a level of from 2% to 4%.

Antidandruff agent

Another essential element of the present compositions is a particulate antidandruff agent. Included among such agents are sulfur, selenium sulfide, salicylic acid, zinc pyridinethione, other 1-hydroxy pyridones, such as those disclosed in U.S.—A—4,185,106 and azole antimycotics disclosed in GB—A—1,502,144 among many others. The antidandruff agent is present at a level of from 0.2% to 4%.

Zinc pyridinethione is the preferred agent, particularly where its salt crystals are predominantly flat platelets which have a mean sphericity less than 0.65, preferably between 0.20 and 0.65, and a median particle diameter of at least $2\mu$, expressed as the diameter of a sphere of equivalent volume. It is preferred that the median particle diameter not be greater than $15\mu$, expressed on the same basis.

The diameter of a sphere of equivalent volume, $d_v$, for a particle can be determined by a variety of sedimentation techniques which are based on Stokes' Law for the settling velocity of a particle in a fluid. Such techniques are described in Stockham, J. D. and Fochtman, E. G., *Particle Size Analysis*, Ann Arbor Science, 1978, incorporated herein by reference.

The sphericity of a particle is also described by Stockham and Fochtman at page 113 as

$$\psi = \left( \frac{d_v}{d_s} \right)^2$$

where $d_v$ is the diameter of a sphere of equivalent volume, supra, and $d_s$ is the diameter of a sphere of equivalent area. A technique for determining $d_s$ is the BET technique described by Stockham and Fochtman at page 122.

Since the sphericity of interest herein is the mean sphericity, the mean diameters are employed.

Water

The compositions of the present invention are lotions with water being the major carrier. The amount of water present is generally from 35% to 90%, preferably from 60% to 80%.

Optional components

The shampoos herein can contain a variety of non-essential optional ingredients suitable for rendering such compositions more stable and desirable. Such conventional optional ingredients are well known to those skilled in the art, e.g., preservatives such as benzyl alcohol, methyl paraben, propyl paraben and imidazoli- dinyl urea; pH adjusting agents such as citric acid, sodium citrate, succinic acid, phosphoric acid, sodium hydroxide, sodium carbonate, etc.; and, sequestering agents such as disodium ethylenediamine tetraacetate.

Minor ingredients such as perfumes, dyes and coloring agents can also be added to the instant compositions to improve their consumer acceptability. If present, such agents generally comprise from 0.1% to 2.0% by weight of the composition.

The shampoos of the present invention are preferably substantially free of suspending agents such as magnesium/aluminum silicate (present at less than 0.1%) and inorganic salts such as alkali metal chlorides and ammonium chloride (present at less than 0.70%). Additionally while the pH may be in the range of from 3 to 9, the preferred pH is from 4 to 6, particularly when ammonium alkyl sulfate is the surfactant. The proper pH is obtained through the use of an appropriate buffer such sodium citrate/citric acid.

The shampoos of the present invention may be made using mixing techniques well known in the art.

The shampoos of the present invention are used in a conventional manner.

The following non-limiting example I further describes and demonstrates an embodiment within the scope of the present invention. Unless otherwise indicated, all percentages herein are by weight.

Example I

A shampoo composition of the present invention having the following composition was prepared.

| Component | | Level |
|---|---|---|
| Ammonium alkyl sulfate (27% aqueous solution) | | 55.25% |
| Citric acid | | 0.24 |
| Sodium citrate | | 0.55 |
| Coconut monoethanolamide | | 3.00 |
| Ethylene glycol distearate | | 5.00 |
| Zinc pyridinethione | | 2.00 |
| Methyl paraben | | 0.20 |
| Propyl paraben | | 0.10 |
| Color solution | | 0.20 |
| Perfume | | 0.50 |
| Water | q.s. | 100.00% |

The composition had a viscosity of about 4 Pa.s (4000 cps) and experienced very little separation after 2 weeks at 48.9°C (120°F).

Example II (comparative)

The following shampoo composition was prepared.

| Component | Level |
| --- | --- |
| Ammonium alkyl sulfate | |
| (27% aqueous solution) | 55.25% |
| Coconut monoethanolamide | 3.0 |
| Ethylene glycol distearate | 5.0 |
| Zinc pyridinethione | 2.0 |
| Ammonium chloride | 1.0 |
| Citric acid | 0.24 |
| Sodium citrate | 0.55 |
| Methyl paraben | 0.20 |
| Propyl paraben | 0.10 |
| Color | 0.15 |
| Perfume | 0.50 |
| Water | q.s. |
| | 100.00 |

This formulation, which contains a level of ammonium chloride outside the scope of the present invention, separated unacceptably after 1 week at 37.8°C (100°F).

Example III (comparative)
The following composition was prepared.

| Component | | Level |
| --- | --- | --- |
| Ammonium alkyl sulfate | | |
| (27% aqueous solution) | | 55.25% |
| Zinc pyridinethione | | 2.00 |
| Coconut monoethanolamide | | 4.64 |
| Ethylene glycol distearate | | 2.35 |
| Citric acid | | 0.20 |
| Methyl paraben | | 0.20 |
| Propyl paraben | | 0.10 |
| Color solution | | 0.35 |
| Perfume | | 0.50 |
| Water | q.s. | 100.00% |

This composition, outside the scope of the present invention, had an acceptable viscosity of 5,520 cps but showed unacceptable liquid separation at 37.8°C (100°F) after 1 month.

## Claims

1. An antidandruff lotion shampoo composition comprising:

(A) a surfactant selected from alkyl sulfates, ethoxylated alkyl sulfates and mixtures thereof;
(B) a suspending agent selected from ethylene glycol esters of fatty acids having from 16 to 22 carbon·atoms, alkanol amides of fatty acids having from 16 to 22 carbon atoms, alkyl ($C_{16-22}$) dimethyl amine oxides and mixtures thereof;
(C) an alkanolamide of a fatty acid having from 8 to 14 carbon atoms;
(D) from 0.2% to 4% of a particulate antidandruff agent; and
(E) from 35% to 90% water,

characterised in that surfactant (A) is from 11% to 20%, suspending agent (B) is from 4% to 6% and alkanolamide (C) is from 2% to 4% of composition and wherein said composition is substantially free of alkali metal chlorides and ammonium chloride.

2. A shampoo composition according to Claim 1 characterised in that the surfactant is selected from ammonium alkyl sulfate, sodium alkyl sulfate, sodium ethoxy (3) alkyl sulfate, triethanolamine alkyl sulfate and mixtures thereof.

3. A shampoo composition according to Claim 1 or 2 characterised in that the suspending agent is selected from ethylene glycol monostearate, ethylene glycol distearate and mixtures thereof.

4. A shampoo composition according to any of Claims 1 to 3 characterised in that the alkanolamide of a fatty acid having from 8 to 14 carbon atoms is selected from coconut monoethanolamide, lauric diethanolamide and mixtures thereof.

5. A shampoo composition according to any of Claims 1 to 4 characterised in that the surfactant is sodium alkyl sulfate wherein the pH of said composition is from 4 to 6.

6. A shampoo composition according to any of Claims 1 to 5 characterised in that the antidandruff agent is zinc pyridinethione.

## Patentansprüche

1. Ein Antischuppen-Haarwaschmittel in Lotionsform, umfassend:

(A) ein oberflächenaktives Mittel, ausgewählt aus Alkylsulfaten, ethoxylierten Alkylsulfaten und Mischungen davon;
(B) ein Suspendiermittel, ausgewählt aus Ethylenglykolestern von Fettsäuren mit 16 bis 22 Kohlenstoffatomen, Alkanolamiden von Fettsäuren mit 16 bis 22 Kohlenstoffatomen, Alkyl($C_{16-22}$)dimethylaminoxiden und Mischungen davon;
(C) ein Alkanolamid einer Fettsäure mit 8 bis 14 Kohlenstoffatomen;
(D) 0,2% bis 4% eines teilchenförmigen Antischuppenmittels; und
(E) 35% bis 90% Wasser,

dadurch gekennzeichnet, daß das oberflächenaktive Mittel (A) 11% bis 20%, das Suspendiermittel (B) 4% bis 6% und das Alkanolamid (C) 2% bis 4% des Mittels ausmacht, und daß das genannte Mittel im wesentlichen von Alkalimetallchloriden und Ammoniumchlorid frei ist.

2. Ein Haarwaschmittel nach Anspruch 1, dadurch gekennzeichnet, daß das oberflächenaktive Mittel aus Ammoniumalkylsulfat, Natriumalkylsulfat, Natriumethoxy(3)alkylsulfat, Triethanolaminalkylsulfat und Mischungen davon ausgewählt ist.

3. Ein Haarwaschmittel nach Anspruch 1 oder 2, dadurch gekennzeichnet, daß das

Suspendiermittel aus Ethylenglykolmono-stearat, Ethylenglykoldistearat und Mischun-gen davon ausgewählt ist.

4. Ein Haarwaschmittel nach irgendeinem der Ansprüche 1 bis 3, dadurch gekennzeichnet, daß das Alkanolamid einer Fettsäure mit 8 bis 14 Kohlenstoffatomen aus Kokosnußmonoethanolamid, Laurinsäurediethanolamid und Mischungen davon ausgewählt ist.

5. Ein Haarwaschmittel nach irgendeinem der Ansprüche 1 bis 4, dadurch gekennzeichnet, daß das oberflächenaktive Mittel Natriumalkylsulfat ist und daß der pH des Haarwaschmittels 4 bis 6 beträgt.

6. Ein Haarwaschmittel nach irgendeinem der Ansprüche 1 bis 5, dadurch gekennzeich-net, daß das Antischuppenmittel Zinkpyridine-thion ist.

**Revendications**

1. Composition de shampooing anti-pelliculaire sous forme de lotion, comprenant:

(A) un tensio-actif choisi parmi les alkyl-sulfates, les alkyl-sulfates éthoxylés et leurs mélanges;
(B) un agent de suspension choisi parmi les esters d'éthylène-glycol et d'acides gras ayant de 16 à 22 atomes de carbone, les alcanol-amides d'acides gras ayant de 16 à 22 atomes de carbone, les oxydes d'alkyl-$(C_{16}—C_{22})$ diméthylamines et leurs mélanges;
(C) un alcanolamide d'un acide gras ayant de 8 à 14 atomes de carbone;

(D) de 0,2% à 4% d'un agent anti-pelliculaire particulaire; et
(E) de 35% à 90% d'eau,

caractérisée en ce que le tensio-actif (A) représente de 11% à 20%, l'agent de suspension (B) représente de 4% à 6% et l'alcanolamide (C) représente de 2% à 4% de la composition, ladite composition étant à peu près exempte de chlorures de métaux alcalins et de chlorure d'ammonium.

2. Composition de shampooing selon la revendication 1, caractérisée en ce que le tensio-actif est choisi parmi un alkyl-sulfate d'ammonium, un alkyl-sulfate de sodium, un éthoxy-3- alkyl-sulfate de sodium, un alkyl-sulfate de triéthanolamine et leurs mélanges.

3. Composition de shampooing selon la revendication 1 ou 2, caractérisée en ce que l'agent de suspension est choisi parmi le monostéarate d'éthylène-glycol, le distéarate d'éthylène-glycol et leurs mélanges.

4. Composition de shampooing selon l'une quelconque des revendications 1 à 3, caractérisée en ce que l'alcanolamide d'un acide gras ayant de 8 à 14 atomes de carbone est choisi parmi le monoéthanolamide de noix de coco, le diéthanolamide laurique et leurs mélanges.

5. Composition de shampooing selon l'une quelconque des revendications 1 à 4, caractérisée en ce que le tensio-actif est un alkyl-sulfate de sodium, le pH de ladite composition se situant entre 4 et 6.

6. Composition de shampooing selon l'une quelconque des revendications 1 à 5, caractérisée en ce que l'agent anti-pelliculaire est de la zinc pyridinethione.